# EUROPEAN PATENT APPLICATION

(11) **EP 3 808 360 A1**
(43) Date of publication of application: **21.04.2021**
(21) Application number: 19830341.4
(22) Date of filing: 02.07.2019
(51) Int. Cl.: A61K 36/899, A61K 35/748, A61K 36/02, A61K 31/409, A23L 33/105

(54) **METHOD FOR PREPARING CHLOROPHYLL-CONTAINING EXTRACT**

(30) Priority: 06.07.2018 KR 20180078919
(71) Applicant: ABL Co.,Ltd, Gwangju 61005 (KR)
(72) Inventor: YEO, Hyoungmin, Gwangju 62246 (KR); RYU, Byung Ju, Gwangju 61903 (KR); IM, Jihwan, Gwangju 62234 (KR); LEE, Myungeun, Gwangju 61268 (KR); CHOI, Chulcuy, Gwangju 61258 (KR)
(74) Representative: ABG Intellectual Property Law, S.L.
(86) International application number: PCT/KR2019/008036
(87) International publication number: WO 2020/009422

(57) **Abstract**

Provided is a method for preparing chlorophyll-containing extract obtained by effectively removing impurities from raw materials and having a high content of chlorophyll.

## Description

### TECHNICAL FIELD

The present invention disclosed herein relates to a method for preparing chlorophyll-containing extract obtained by effectively removing impurities from raw materials and having a high content of chlorophyll.

### BACKGROUND ART

Unlike synthetic pigments which have toxicity and carcinogenicity, natural pigments derived from food have a variety of physiologically active ingredients such as vitamins, minerals, and polyphenol compounds, and are also safe, so the preference and demand therefor are increasing. Chlorophyll, which is a natural pigment obtainable from vegetables and fruits and widely used in the food industry, has a form in which magnesium (Mg) ions are bonded to the center of a porphyrin ring, and may be classified into types such as chlorophyll a, b, c, and d. In addition, there is a difference in the content of chlorophyll depending on the variety, cultivation soil, and harvest time of a plant from which the chlorophyll is extracted, and the content thereof is changed by heat, light, oxygen, acids, enzymes, and the like, so that chlorophyll has a disadvantage in terms of quality and customer satisfaction.

Chlorophyll and chlorophyll derivatives show a variety of physiological activities, and thus, have been studied in various fields such as wound therapy, antiinflammatory treatment and anticancer therapy. Among the chlorophyll derivatives, chlorine e-6 plays an important role as a photosensitizer in photodynamic therapy, and copper chlorophyllin sodium is also used for the purpose of removing body odor, treating inflammation, and the like.

Types of such chlorophyll derivatives may be divided into pheophytin and pyrophetin series in which a magnesium metal in the center has been substituted or removed, chlorophyllide and pheophorbide series in which a magnesium metal and phytol are removed, and chlorine series produced through saponification reaction.

In 1960, a synthesis team of 17 post-doctoral researchers led by Professor Woodward successfully synthesized chlorophyll a. However, the process is very complicated and long, and thus, is not suitable for commercial use.

As a typical method for extracting chlorophyll in plants, there is a method of performing extraction using various organic solvents such as acetone, ethanol, hexane, and dioxane, and then removing impurities through centrifugation, low-temperature immersion, and the like. However, there is a problem in that the method uses a solvent that should not be used for food and medicine. Thus, in order to solve the above problem, there have been efforts to use and apply ethanol to food and the like. However, there is a problem in that an excessive amount of ethanol is used in an impurities removal step or an extraction step. Also, there is still a problem in that an organic solvent such as acetone is used to remove polar impurities after extraction.

Meanwhile, extraction using a supercritical fluids has advantages such as high solubility, fast mass transfer and heat transfer, low viscosity, a high diffusion rate, and fast permeability to micro-pores due to low surface tension, and is it an extraction method which may solve technical difficulties such as low efficiency, low quality, low speed, and adverse impact on the environment in existing processes such as reactions, decomposition, extraction, distillation, crystallization, absorption, adsorption, drying, and washing.

Supercritical fluid extraction may fundamentally prevent or minimize the generation of various pollution sources (air, water, and land) in a product production process, and may also fundamentally prevent harmfulness to human health and the environment, and thus, may bring economic benefits compared to conventional product production technologies.

Korean Patent Registration No. 1428486 discloses a method for preparing chlorella extract using a supercritical fluid extraction method, but the extraction target and extraction method thereof are different from those of a method for preparing chlorophyll-containing extract using a supercritical fluid extraction method of the present invention.

### DISCLOSURE OF THE INVENTION

### TECHNICAL PROBLEM

The present invention is to solve the problems of a typical organic solvent extraction method as described above, and to provide a method for preparing chlorophyll-containing extract, in which impurities are effectively removed with a relatively simple and easy method, and the extraction efficiency of chlorophyll is increased as well.

### TECHNICAL SOLUTION

In accordance with an embodiment of the present invention to achieve the above object, a method for preparing chlorophyll-containing extract includes an chlorophyll extraction step of performing complex supercritical extraction on a plant or microorganism containing chlorophyll.

Also, the plant or microorganism may be selected from the group consisting of woody leaves, woody buds, herbal stems, herbal leaves, herbal buds; cyanophyta, prochlorophyta, rhodophyta, chlorophyta, heterokontophyta, tribophyta, glaucophyta, chlorarachniophytes, euglenophyta, euglenoids, haptophyta, chrysophyta, cryptophyta, cryptomonads, dinophyta, dinoflagellata, pyrmnesiophyta, bacillariophyta, xanthophyta, eustigmatophyta, raphidophyta, phaeophyta, phytoplankton; blue-green bacteria such as spirulina; microalgae such as Chlorella genus, Scenedesmus genus, Dunaliella genus, Chlamydomonas genus, Haematococcus genus, and Botryococcus genus; and a combination thereof.

In addition, in the chlorophyll extraction step, a main solvent may be carbon dioxide, and a co-solvent may be ethanol.

Also, in the chlorophyll extraction step, the total flow rate of the complex supercritical extraction may be 20 to 120 mℓ /min, preferably 30 to 100 mℓ /min, and more preferably 40 to 85 mℓ /min.

Also, in the chlorophyll extraction step, the flow rate of the co-solvent of the complex supercritical extraction may be 1 to 20 mℓ /min, preferably 2 to 15 mℓ /min, and more preferably 3 to 12 mℓ /min.

Also, in the chlorophyll extraction step, the temperature of the complex supercritical extraction may be 30 to 70°C, preferably 40 to 60°C, and more preferably 45 to 55°C.

Also, in the chlorophyll extraction step, the pressure of the complex supercritical extraction may be 70 to 650 bar, preferably 100 to 500 bar, and more preferably 150 to 400 bar.

Also, in the chlorophyll extraction step, the duration of the complex supercritical extraction may be 45 to 300 minutes, preferably 60 to 250 minutes, and more preferably 70 to 220 minutes.

Also, the method for preparing chlorophyll-containing extract of the present invention may further include, before the chlorophyll extraction step, an impurities removal step of performing simple supercritical extraction on a plant or a microorganism.

Also, in the impurities removal step, the solvent of the simple supercritical extraction may be carbon dioxide.

Also, in the impurities removal step, the flow rate of the solvent of the simple supercritical extraction may be 20 to 120 mℓ /min, preferably 30 to 100 mℓ /min, and more preferably 40 to 85 mℓ /min.

Also, in the impurities removal step, the temperature of the simple supercritical extraction may be 30 to 70°C, preferably 40 to 60°C, and more preferably 45 to 55°C.

Also, in the impurities removal step, the pressure of the simple supercritical extraction may be 70 to 650 bar, preferably 100 to 500 bar, and more preferably 150 to 400 bar.

Also, in the impurities removal step, the duration of the simple supercritical extraction may be 30 to 350 minutes, preferably 40 to 300 minutes, and more preferably 50 to 250 minutes.

Also, the method for preparing chlorophyll-containing extract of the present invention may further include, before the impurities removal step, a pre-treatment step of mixing a plant or a microorganism with oil.

Also, the oil of the pre-treatment step may be vegetable oil.

Also, the oil of the pre-treatment step may be, preferably, oil selected from the group consisting of soybean oil, canola oil, sunflower seed oil, macadamia oil, peanut oil, grapeseed oil, pumpkin seed oil, flaxseed oil, linseed oil, olive oil, corn oil, safflower oil, sesame oil, almond oil, peach seed oil, apricot seed oil, walnut oil, rapeseed oil, raspberry oil, bilberry seed oil, cranberry seed oil, pomegranate seed oil, sacha inchi oil, fruit seed oil, sea buckthorn seed oil, chia oil, perilla oil; conjugated linolenic acid; diacylglycerol oil; omega 3 fatty acid, or a mixture thereof.

Also, in the pre-treatment step, the weight ratio of the oil to 100 parts by weight of the plant or the microorganism may be 1 to 50 parts by weight, preferably 2 to 10 parts by weight.

Also, the method for preparing chlorophyll-containing extract of the present invention may further include, before the pre-treatment step, a powdering step of freeze-drying and then pulverizing a plant or a microorganism.

Also, an operating temperature coefficient may be 0.7 to 1.15, preferably 0.75 to 1.1, and more preferably 0.9 to 0.97.

### ADVANTAGEOUS EFFECTS

Chlorophyll-containing extract prepared through a complex supercritical fluid extraction process of the present invention has no organic solvent introduced during a preparation process, and thus, may be applied directly to food or medicine without any additional post-treatment. Also, an organic solvent removal process is not needed, and thus, there is an effect of saving installation cost and operation cost required therefor. In addition, since raw materials are extracted using only the carbon dioxide before the complex supercritical fluid extraction process in which ethanol is added, impurities in the raw materials may be effectively removed. Furthermore, the method for preparing chlorophyll-containing extract of the present invention has an advantage of further increasing the content of chlorophyll in the chlorophyll-containing extract by performing pre-treatment on raw materials with oil when removing the impurities.

### BRIEF DESCRIPTION OF THE DRAWINGS

FIG. 1 is a photograph of wheatgrass powder before (A) and after (B) the chlorophyll extraction step of the present invention; and
FIG. 2 is an HPLC result for analyzing the content of chlorophyll in an extract after the supercritical extraction of wheatgrass powder {(A) Comparative Example 1, (B) Example 1, (C) Comparative Example 2, (D) Example 2 [(a) chlorophyll a, (a') chlorophyll a epimer, (b) chlorophyll b, (b') chlorophyll b epimer, (c) carotenoid] } .

### BEST MODE FOR CARRYING OUT THE INVENTION

Hereinafter, preferred embodiments of the present invention will be described in detail.

However, below is only to describe specific embodiments in detail. The present invention may be changed in various ways and may have a number of forms, and thus, the present invention is not limited to specific embodiments illustrated. It is to be understood that the present invention includes all changes, equivalents, and alternatives falling within the spirit and scope of the present invention.

In addition, in the following description, many specific details such as specific components are described. However, they are only provided to aid in further understanding of the present invention, and thus, it will be apparent to those skilled in the art that the present invention may be practiced without these specific details. Also, in describing the present invention, detailed descriptions of related known functions or configurations will be omitted when it is determined that the detailed descriptions may unnecessarily obscure the gist of the present invention.

Also, the terms used in the present application are only used to describe specific embodiments, and are not intended to limit the present invention. Unless defined otherwise, all the terms used herein, including technical or scientific terms, may have the same meanings as those commonly understood by those skilled in the art to which the present invention pertains. Terms that are defined in a commonly used dictionary should be construed as having meanings consistent with the meanings in the context of the related art, and should meaning not be construed as having an ideal or overly formal meaning unless explicitly defined in the present application.

In the present application, the terms of a singular form may include a plural form unless the context clearly indicates otherwise.

In the present application, terms such as 'include,' 'contain,' or 'have' are intended to refer to the presence of features, components (or elements), and the like described in the specification, and do not imply that one or more other features or components are not present or cannot be added.

In the present application, the operating temperature coefficient is defined as follows (all temperature units are absolute temperature K):

Operating temperature coefficient = (Freeze-drying temperature / Boiling point of co-solvent in chlorophyll extraction step) × (Boiling point (low point) of oil in pre-treatment step / Critical temperature of solvent in impurities removal step).

The method for preparing chlorophyll-containing extract of the present invention includes an chlorophyll extraction step of performing complex supercritical extraction on a plant or a microorganism containing chlorophyll. Here, complex supercritical extraction refers to extracting in combination of a co-solvent with a main solvent, instead of using the main solvent alone.

The plant or the microorganism used as a raw material for extraction is not limited as long as it is a plant or a microorganism including chlorophyll a, chlorophyll b, chlorophyll c1, chlorophyll c2, chlorophyll d, chlorophyll f, bacteriochlorophyll, or a mixture thereof. For example, the plant or the microorganism may be selected from the group consisting of woody leaves, woody buds, herbal stems, herbal leaves, herbal buds; cyanophyta, prochlorophyta, rhodophyta, chlorophyta, heterokontophyta, tribophyta, glaucophyta, chlorarachniophytes, euglenophyta, euglenoids, haptophyta, chrysophyta, cryptophyta, cryptomonads, dinophyta, dinoflagellata, pyrmnesiophyta, bacillariophyta, xanthophyta, eustigmatophyta, raphidophyta, phaeophyta, phytoplankton; blue-green bacteria such as spirulina; microalgae such as Chlorella genus, Scenedesmus genus, Dunaliella genus, Chlamydomonas genus, Haematococcus genus, and Botryococcus genus; and a combination thereof.

In the chlorophyll extraction step, a main solvent may be carbon dioxide, and a co-solvent may be ethanol.

In the chlorophyll extraction step, the total flow rate of the complex supercritical extraction may be 20 to 120 mℓ /min, preferably 30 to 100 mℓ /min, and more preferably 40 to 85 mℓ /min. When the total flow rate is less than the above range, there is a problem in that extraction duration becomes longer. On the contrary, when exceeding the above range, the extraction duration becomes shorter, but there is a problem in that a supercritical state may not be maintained during the process.

In the chlorophyll extraction step, the flow rate of the co-solvent of the complex supercritical extraction may be 1 to 20 mℓ /min, preferably 2 to 15 mℓ /min, and more preferably 3 to 12 mℓ /min. When the flow rate of the co-solvent is less than the above range, there is a problem in that extraction duration becomes longer. On the contrary, when exceeding the above range, the extraction duration becomes shorter, but there is a problem in that a supercritical state may not be maintained during the process.

In the chlorophyll extraction step, the temperature of the complex supercritical extraction may be 30 to 70°C, preferably 40 to 60°C, and more preferably 45 to 55°C. When the temperature of the complex supercritical extraction is less than the above range, there is a problem in that the chlorophyll extraction efficiency decreases. On the contrary, when exceeding the above range, there is a problem in that impurities are extracted together.

In the chlorophyll extraction step, the pressure of the complex supercritical extraction may be 70 to 650 bar, preferably 100 to 500 bar, and more preferably 150 to 400 bar. When the pressure of the complex supercritical extraction is less than the above range, there is a problem in that the chlorophyll extraction efficiency decreases. On the contrary, when exceeding the above range, there is a problem in that impurities are extracted together.

In the chlorophyll extraction step, the duration of the complex supercritical extraction may be 45 to 300 minutes, preferably 60 to 250 minutes, and more preferably 70 to 220 minutes. When the duration of the complex supercritical extraction is less than the above range, there is a problem in that chlorophyll is not completely extracted so that some chlorophyll remains in raw materials. On the contrary, when exceeding the above range, since all chlorophyll is already extracted, there is no chlorophyll left to be extracted in a later stage of the extraction, so that there is a problem in that economic feasibility decreases.

The method for preparing chlorophyll-containing extract of the present invention may further include, before the chlorophyll extraction step, an impurities removal step of performing simple supercritical extraction on a plant or a microorganism. Here, the simple supercritical extraction refers to extracting using a main solvent alone. It is the main feature of the present invention that impurities are extracted first using the main solvent alone as described above before extracting chlorophyll in combination of the main solvent and a co-solvent.

In the impurities removal step, the solvent of the simple supercritical extraction may be carbon dioxide.

In the impurities removal step, the flow rate of the solvent of the simple supercritical extraction may be 20 to 120 mℓ /min, preferably 30 to 100 mℓ /min, and more preferably 40 to 85 mℓ /min. When the flow rate of the co-solvent of the simple supercritical extraction is less than the above range, there is a problem in that extraction duration becomes longer. On the contrary, when exceeding the above range, the extraction duration becomes shorter, but there is a problem in that a supercritical state may not be maintained during the process.

In the impurities removal step, the temperature of the simple supercritical extraction may be 30 to 70°C, preferably 40 to 60°C, and more preferably 45 to 55°C. When the temperature of the simple supercritical extraction is less than the above range, there is a problem in that impurities are not completely removed. On the contrary, when exceeding the above range, there is a problem in that some of chlorophyll is removed together with the impurities.

In the impurities removal step, the pressure of the simple supercritical extraction may be 70 to 650 bar, preferably 100 to 500 bar, and more preferably 150 to 400 bar. When the pressure of the simple supercritical extraction is less than the above range, there is a problem in that impurities are not completely removed. On the contrary, when exceeding the above range, there is a problem in that some of chlorophyll is removed together with the impurities.

In the impurities removal step, the duration of the simple supercritical extraction may be 30 to 350 minutes, preferably 40 to 300 minutes, and more preferably 50 to 250 minutes. When the duration of the simple supercritical extraction is less than the above range, there is a problem in that impurities are not completely extracted so that some impurities remain in raw materials. On the contrary, when exceeding the above range, since all impurities are already extracted, there are no impurities left to be extracted in a later stage of the extraction, so that there is a problem in that economic feasibility decreases and some chlorophyll is removed.

The method for preparing chlorophyll-containing extract of the present invention may further include, before the impurities removal step, a pre-treatment step of mixing a plant or a microorganism with oil. When raw material powder is mixed with oil, impurities in raw materials are dissolved in the oil. As a result, the efficiency of removing impurities in the raw materials is significantly increased in the impurities removal step, and this pre-treatment step constitutes another major feature of the present invention.

The oil of the pre-treatment step may be vegetable oil.

The oil of the pre-treatment step may be, preferably, oil selected from the group consisting of soybean oil, canola oil, sunflower seed oil, macadamia oil, peanut oil, grapeseed oil, pumpkin seed oil, flaxseed oil, linseed oil, olive oil, corn oil, safflower oil, sesame oil, almond oil, peach seed oil, apricot seed oil, walnut oil, rapeseed oil, raspberry oil, bilberry seed oil, cranberry seed oil, pomegranate seed oil, sacha inchi oil, fruit seed oil, sea buckthorn seed oil, chia oil, perilla oil; conjugated linolenic acid; diacylglycerol oil; omega 3 fatty acid, or a mixture thereof.

In the pre-treatment step, the weight ratio of the oil to 100 parts by weight of the plant or the microorganism may be 1 to 50 parts by weight, preferably 2 to 10 parts by weight. When the weight ratio of the oil to the raw materials is less than the above range, there is a problem in that impurities are not completely removed. On the contrary, when exceeding the above range, there is a problem in that some of chlorophyll is removed together with the impurities.

The method for preparing chlorophyll-containing extract of the present invention may further include, before the pre-treatment step, a powdering step of freeze-drying and then pulverizing a plant or a microorganism.

Since the present invention is operated under severe conditions of supercritical extraction other than room temperature and atmospheric pressure, it is necessary to design a process so that the operating cost does not increase rapidly. Among the above, processing temperatures, which are important, may include the freeze-drying temperature, the boiling point of the co-solvent in the chlorophyll extraction step, the boiling point of the oil in the pre-treatment step, and the critical temperature of the solvent in the impurities removal step. Among the above, the higher the freeze-drying temperature, the lower the operating cost. The lower the boiling point of the co-solvent in the chlorophyll extraction step, the lower the operating cost. The higher the boiling point of the oil in the pre-treatment step, the easier the mixing with the raw material powder. The lower the critical temperature of the solvent in the impurities removal step, the lower the operating cost.

With this concept in mind, the present invention has introduced a new operating variable called the operating temperature coefficient, and the operating temperature coefficient is defined as follows (all temperature units are absolute temperature K):

Operating temperature coefficient = (Freeze-drying temperature / Boiling point of co-solvent in chlorophyll extraction step) × (Boiling point (low point) of oil in pre-treatment step / Critical temperature of solvent in impurities removal step).

After reviewing various materials, it has been found that an operating temperature coefficient may be 0.7 to 1.15, preferably 0.75 to 1.1, and more preferably 0.9 to 0.97. When the operating temperature coefficient is less than the above range, there is a disadvantage in that the operating cost increases excessively. On the contrary, when exceeding the above range, the operating cost decreases, but there is a problem in that the material cost increases due to the use of expensive materials.

Using chlorophyll obtained by the above method, it was possible to obtain a variety of chlorophyll derivatives derived from the chlorophyll through a method known in the art.

Hereinafter, examples of the present invention will be described in detail. However, the following examples are illustrative of the present invention, and the present invention is not limited by the following examples.

### Examples

### Test Example: Measuring mass of chlorophyll a

In order to analyze the content of extracted chlorophyll, high-performance liquid chromatography (AGILENT, USA) was used, and the column used was Shimadzu Shim-pack C18 analytical column (250 mm x 4.6 mm, 5 µm, 100). The peak was detected as ultraviolet ray absorption using a diode array detector. The injection volume was 20 µℓ, and the flow rate was 1 mℓ/min. The reference material for chlorophyll a was purchased from Aldrich Chemical Co. (Milwaukee, WI), and was used without a further purification process.

### Comparative Example 1: Alcohol extraction step

1 g of freeze-dried powder wheatgrass (Dreams, South Korea) was added to 25 mℓ of ethanol (98%), stirred for 2 hours at room temperature to be extracted, and then filtered using a filter paper. Thereafter, the filtrate was analyzed according to Test Example above to obtain the content of chlorophyll a. According to the analysis result, the amount of chlorophyll a was 3.8 mg, and the mass of total solids remaining after ethanol was removed by vacuum distillation was confirmed to be 165 mg.

### Example 1: Supercritical extraction step

50 g of freeze-dried powder wheatgrass (Dreams, South Korea) was extracted for 60 minutes under the conditions of 350 bar, 50°C, and a supercritical carbon dioxide flow rate of 60 mℓ/min to remove impurities, and then extracted again for 120 minutes under the conditions of 350 bar, 50°C, a supercritical carbon dioxide flow rate of 60 mℓ/min, and a co-solvent ethanol flow rate of 5 mℓ/min to analyze the content of chlorophyll a. According to the analysis result, the amount of chlorophyll a was 235 mg, and the mass of total solids remaining after ethanol was removed by vacuum distillation was confirmed to be 4250 mg.

### Preparation Example 1: Pre-treatment step + Impurities removal step

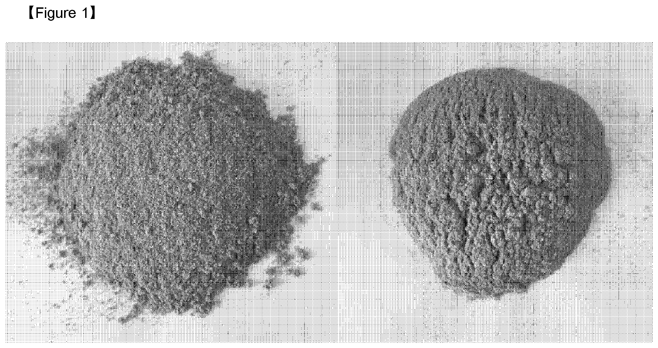

50 g of freeze-dried wheatgrass powder (Dreams, South Korea) and 2.5 g of grape seed oil as shown in (A) of FIG. 1 were mixed well with a mixer to be prepared. The prepared mixture of wheatgrass and grape seed oil was extracted for 120 minutes under the conditions of 350 bar, 50°C, and a supercritical carbon dioxide flow rate of 60 mℓ/min to obtain wheatgrass powder with improved clarity of green color by removing impurities as shown in (B) of FIG. 1.

### Comparative Example 2: Preparation Example 1 + Comparative Example 1

The content of chlorophyll a obtained through the step of Comparative Example 1 was analyzed with respect to the wheatgrass powder obtained through the steps of Preparation Example 1. According to the analysis result, the amount of chlorophyll a was 4.2 mg, and the mass of total solids remaining after ethanol was removed by vacuum distillation was confirmed to be 87.5 mg.

### Example 2: Preparation Example 1 + Example 1

The content of chlorophyll a obtained through the step of Example 1 was analyzed with respect to the wheatgrass powder obtained through the steps of Preparation Example 1. According to the analysis result, the amount of chlorophyll a was 240 mg, and the mass of total solids remaining after ethanol was removed by vacuum distillation was confirmed to be 3116 mg.

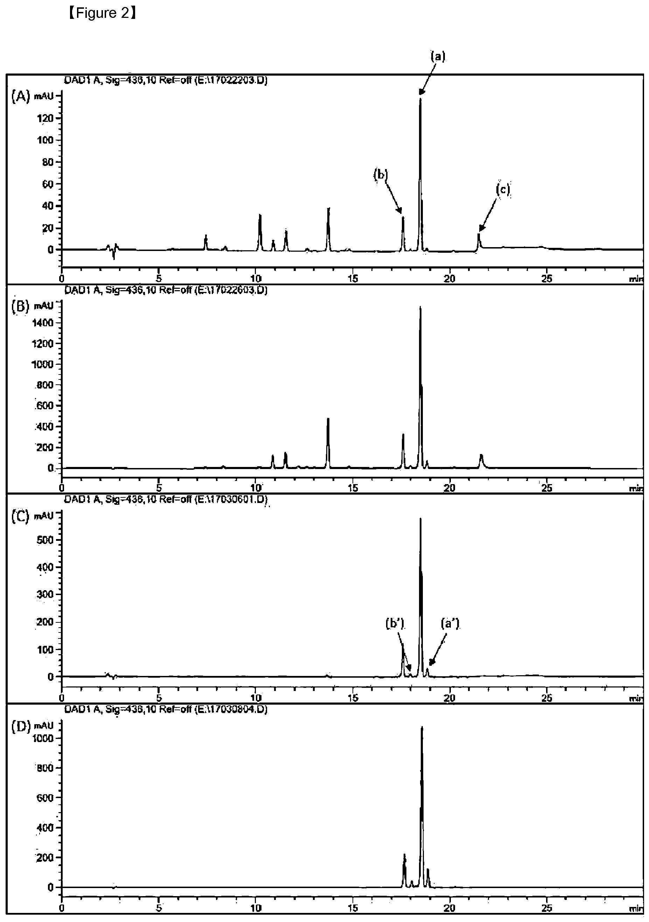

The result of converting the amount of chlorophyll a analyzed in each of Comparative Examples 1 and 2 and Examples 1 and 2 with respect to 1 g of dry wheatgrass powder and the extraction efficiency (= mass of chlorophyll a / mass of total solids) are shown in Table 1 below. Also, the results of high-performance liquid chromatography of each thereof are shown in FIG. 2.

**[Table 1]**

| Wheatgrass powder | Comparative Example 1 | Example 1 | Comparative Example 2 | Example 2 |
|---|---|---|---|---|
| Chlorophyll (mg) | 3.8 | 4.7 | 4.2 | 4.8 |
| Extract efficiency (%) | 2.3 | 5.5 | 4.8 | 7.2 |

According to FIG. 2, a characteristic peak of chlorophyll b and a characteristic peak of chlorophyll a were observed in the 17 minute and 18 minute, respectively, and in addition, a characteristic peak of carotenoid was observed in the 22 minute.

In the drawing of Comparative Example 1 (FIG 2 (A)), various impurity peaks were observed before the 15 minute, and in the drawing of Example 1 (FIG. 2(B)), it was confirmed that some impurities were removed.

In the case of Comparative Example 2 (FIG. 2 (C)) and Example 2 (FIG. 2 (D)), it was confirmed that most of the impurities were removed. However, after the removal of the impurities, it was confirmed that the chlorophyll content and extraction efficiency of supercritical extraction in which a co-solvent was used were better than those of alcohol extraction.

### Comparative Examples 3,4 to Example 3,4: Barley sprout powder

The same procedures as in Comparative Examples 1 and 2 and Examples 1 and 2 were performed, but instead of wheatgrass powder, barley sprout powder (Dreams Co., Ltd., South Korea) was used. The result of converting the analyzed amount of chlorophyll a with respect to 1 g of dry barley sprout powder and the extraction efficiency (= mass of chlorophyll a / mass of total solids) are shown in Table 2 below.

**[Table 2]**

| Barley sprout powder | Comparative Example 3 | Example 3 | Comparative Example 4 | Example 4 |
|---|---|---|---|---|
| Chlorophyll (mg) | 3.75 | 4.3 | 4.1 | 4.7 |
| Extract efficiency (%) | 2.5 | 4 | 3.2 | 6 |

### Comparative Examples 5,6 to Example 5,6: Spirulina powder

The same procedures as in Comparative Examples 1 and 2 and Examples 1 and 2 were performed, but instead of wheatgrass powder, spirulina powder (NutriVita Shop, USA) was used. The result of converting the analyzed amount of chlorophyll a with respect to 1 g of dry spirulina powder and the extraction efficiency (= mass of chlorophyll a / mass of total solids) are shown in Table 3 below.

**[Table 3]**

| Spirulina powder | Comparative Example 5 | Example 5 | Comparative Example 6 | Example 6 |
|---|---|---|---|---|
| Chlorophyll (mg) | 2.8 | 3.2 | 2.8 | 4 |
| Extract efficiency (%) | 1.9 | 3.5 | 3.1 | 4.7 |

Although the preferred embodiments of the present invention have been described above, the present invention is not limited to the specific embodiments described above. Various modifications may be implemented by those skilled in the art without departing from the gist of the present invention. Therefore, the scope of the present invention should not be limited to the embodiments described above, but should be defined by the following claims as well as the equivalents thereof.

## Claims

1. A method for preparing chlorophyll-containing extract, the method comprising a chlorophyll extraction step of performing complex supercritical extraction on a plant or microorganism containing chlorophyll.

2. The method of claim 1, wherein the plant or microorganism is selected from the group consisting of woody leaves, woody buds, herbal stems, herbal leaves, herbal buds; cyanophyta, prochlorophyta, rhodophyta, chlorophyta, heterokontophyta, tribophyta, glaucophyta, chlorarachniophytes, euglenophyta, euglenoids, haptophyta, chrysophyta, cryptophyta, cryptomonads, dinophyta, dinoflagellata, pyrmnesiophyta, bacillariophyta, xanthophyta, eustigmatophyta, raphidophyta, phaeophyta, phytoplankton; blue-green bacteria such as spirulina; microalgae such as Chlorella genus, Scenedesmus genus, Dunaliella genus, Chlamydomonas genus, Haematococcus genus, and Botryococcus genus; and a combination thereof.

3. The method of claim 1, wherein, in the chlorophyll extraction step, a main solvent is carbon dioxide, and a co-solvent is ethanol.
